# EUROPEAN PATENT SPECIFICATION

(11) **EP 2 635 213 B1**
(45) Date of publication and mention of the grant of the patent: **27.06.2018**
(21) Application number: 11838358.7
(22) Date of filing: 03.11.2011
(51) Int. Cl.: A61B 17/88, A61B 17/70

(54) **STABILIZERS, END CAP AND CONNECTOR FOR ASSISTING STABILIZATION OF A SPINAL IMPLANT**
STABILISATOREN, ENDKAPPE UND VERBINDER ZUR UNTERSTÜTZUNG DER STABILISIERUNG EINES WIRBELSÄULENIMPLANTATS
STABILISATEURS, CAPUCHON D'EXTRÉMITÉ ET CONNECTEUR FACILITANT LA STABILISATION D'UN IMPLANT RACHIDIEN

(30) Priority: 06.11.2010 US 927050; 30.08.2011 US 201113199418
(43) Date of publication of application: 11.09.2013
(73) Proprietor: K2M, Inc., Leesburg, VA 20175 (US)
(72) Inventor: CASTRO, Frank, Louisville, KY 40245 (US)
(74) Representative: Siekmann, Gunnar
(86) International application number: PCT/US2011/001852
(87) International publication number: WO 2012/060877

(56) References cited:
- EP-A2- 1 358 861
- WO-A1-2011/028236
- GB-A- 2 364 643
- US-A1- 2004 153 160
- US-A1- 2007 123 987
- US-A1- 2007 255 409
- US-A1- 2009 036 985
- US-B1- 7 435 261
- US-B1- 7 435 261
- US-B2- 7 641 701

## Description

### BACKGROUND OF THE INVENTION

### 1. Field of the Invention

Among other things, select preferred embodiments of the present invention are related to a surgical implant or a biocompatible cage and end cap combination or biocompatible cage end cap and connector combination that can be inserted into a cavity that has been created by removing spinal tissue. Post operative and prior to complete arthrodesis, the current invention inhibits extrusion of the cage against the spinal cord. Inclusion of a connector between the biocompatible implant and the end cap allows the spinal column to be distracted a greater length than the length of the biocompatible implant.

Examples are related to a stabilizer for a spinal implant. Select preferred embodiments of the current stabilizer have a gap for creating a socket for receiving a lengthwise end of the spinal implant. In the practice of the present stabilizer, the stabilizer's body is fastened to bone and includes a cover attachable to the body that blocks egress of the spinal implant from the socket as well egress of the fasteners securing the stabilizer's body to bone. Preferred embodiments of stabilizers are manufactured in many geometric shapes and can be utilized with a plethora of spinal implants.

### B. Description of the Previous Art

Any discussion of references cited in this Description of the Previous Art merely summarizes the disclosures of the cited references and Applicant makes no admission that any cited reference or portion thereof is relevant prior art. Applicant reserves the right to challenge the accuracy, relevancy and veracity of the cited references.

Applicant is aware of the following references: 1) US Patent No. 5609635-Michelson enables a lordotic interbody spinal fusion implant; 2) US Patent No. 6491724-Ferree enables a spinal fusion cage with lordosis correction; 3) US Patent No. 6576016-Hochshuler, et al. enables an adjustable height fusion device; 4) US Published Patent Application No. 20050071006-Kirschman discloses a spinal fusion system and method for fusing spinal bones; 5) US Patent 7182782-Kirschman enables methods for adjustable bone fusion implants; 6) US Published Patent Application 20070129805-Braddock Jr., et al. teaches an end device for a vertebral implant; 7) US Patent 6776798-Camino, et al. enables a spacer assembly for use in spinal surgeries having end cap which includes serrated surface; 8) US Published Patent Application 20070255413-Edie, et al. teaches expandable intervertebral spacers and methods of use; 9) US Patent 6808538-Papponeau enables a vertebral body spacer having variable wedged endplates; 10) US Published Patent Application 20040073314-White, et al. teaches vertebral body and disc space devices; 11) US Patent No. 6582432-Michelson enables a cap for use with artificial spinal fusion implant; 12) US Patent No. 5980522-Koros, et al. enables expandable spinal implants; 13) US Patent No. 5458638-Kuslich, et al. enables a non-threaded spinal implant; 14) US Patent 4961740-Ray, et al. enables a V-thread fusion cage and method of fusing a bone joint; 15) US Pub. Patent Application 20080058939-Hughes, et al. discloses a revision spacer; 16) US Pub. Patent Application 20090138083-Biyani discloses a variable height vertebral body replacement implant; 17) US Patents Nos. 7435261-Castro, 7942932-Castro, 8002832-Castro and 8016887-Castro, owned by Applicant, enable spinal implants; 18) US Pub. Patent Application 2007/123987-Bernstein, et al. discloses an interbody spacer assembly including a pair of end pieces and having ratchets to connect the two end pieces; and 19) International Patent Application WO 2011/028236-Castro, owned by Applicant, discloses a spinal implant having a wedge-like cage, which assists the surgical team in not impinging the spinal cord with the spinal implant.

Regarding the references identified in this Description of the Previous Art section, among other things, it does not appear that any of the inventions disclosed at least in the references 1 to 17 practices the use of a cage having a trapezoidal divider, an end cap with docking slides and plate including an opening, a connector positioned between the cage and the end cap or a socket for assisting stabilization of a spinal implant where the socket comprises a polygonal, cylindrical or elliptic cylindrical body surrounding a cavity, where the body comprises a gap at a first end that creates the socket, an extension that extends beyond a second end for receiving fasteners and a cover attachable to the extension for blocking egress of the spinal implant from the socket.

### SUMMARY OF THE INVENTION

Unlike traditional spinal implants, preferred embodiments of the present invention provide a biocompatible cage that assists the surgical team in not impinging the spinal cord with the implant. Post operative and prior to complete arthrodesis, the generally wedge-like implant inhibits extrusion of the cage against the spinal cord. Utilization of an end cap with the biocompatible implant supplements stabilization of the biocompatible implant. And inclusion of a connector positioned between the biocompatible implant and the end cap allows the spinal column to be distracted to a greater distance than the length of the biocompatible implant.

The present invention also provides preferred embodiments of a stabilizer capable of assisting the stabilization of the spinal implant after the spinal implant is inserted into a surgically created cavity. Preferred embodiments of the current stabilizer include a body that is secured to bone. Within the scope of the present invention, the stabilizer body includes a gap that creates a socket for receiving a lengthwise edge of the spinal implant. In accord with the present invention, sockets can be manufactured in different shapes to accommodate the different shapes of the spinal implants. And a cover is attachable to the body to block egress, among other things, of the spinal implant from the socket.

An aspect of the present invention is to provide a biocompatible cage. Still another aspect of the present invention is to provide a generally wedge-like cage. It is still another aspect of the present invention to provide an implant having select embodiments that can be implanted through the patient's frontal or rearward side. Yet still another aspect of the present invention is to provide a cage that can be severed across a first cross-section or severed across a first cross-section and a second cross-section to create a custom fitted implant for the surgically created cavity. Still another aspect of the present invention is to provide a biocompatible cage including brakes. Yet another aspect of the present invention is to provide an end cap attachable to vertebra for assisting with the stabilization of the spinal implant. It is still another aspect of the present invention to provide a connector capable of extending the length of the spinal implant. Still another aspect of the present invention is to provide a spinal implant capable of enhancing lordosis. Yet another aspect of the present invention is to provide a biocompatible implant and end cap combination. It is still another aspect of the present invention to provide a biocompatible, end cap and connector combination. An aspect of the present invention is to provide a stabilizer for assisting stabilization of a spinal implant. Still another aspect of the present invention is to provide a stabilizer including a gap therein for creating a socket for receiving a coupling end of the spinal implantYet still another aspect of the present invention is to provide stabilizers with trapezoidal and optional rectangular and/or hexagonal bodies capable of stabilizing amorphous or geometrically defined spinal implant supporting structures. It is still another aspect of a preferred embodiment of the present invention to provide a body including an extension that has a plurality of apertures. Still another aspect of the present invention is to provide a cover including an aperture for aligning with one of the plurality of apertures located in the body's extension. It is another aspect of the present invention to provide an embodiment that utilizes a fastener to secure the cover to the body.

It is the novel and unique interaction of these simple elements which creates the spinal implants, within the ambit of the present invention. Descriptions of preferred embodiments follow. However, it is to be understood that the best mode descriptions do not limit the scope of the present invention.

### BRIEF DESCRIPTION OF THE DRAWINGS

FIG. 1 is an exploded perspective of an example enabling a biocompatible implant and end cap combination (3000, 4000).
FIG. 2 is an exploded perspective of a preferred embodiment enabling a biocompatible implant and end cap combination (3000, 4000).
FIG. 3 is a perspective of a preferred embodiment of an implant facing side of a border (4010) of an end cap (4000) within the scope of the present invention.
FIG. 4 is a perspective of a preferred embodiment of a connector (4500) capable of being positioned between the implant (3000) and the end cap (4000).
FIG. 5 is a plan view of a preferred embodiment of the coplanar margin of a connector (4500).
FIG. 6 is an exploded perspective of a preferred embodiment of the combination of a biocompatible cage (3000), a connector (4500) and an end cap (4000).
FIG. 7 is a perspective of a preferred embodiment of an engaged combination of the biocompatible cage (3000), connector (4500) or extension and end cap (4000),
FIG. 8 is an exploded view frontal perspective of an example of the stabilizer (20) having a polygonal body (30) and attachable cover (60).
FIG. 9 is a frontal view of an example embodiment of stabilizer (20) where cover (60) is attached to anterior wall (42) of polygonal body (30).
FIG. 10 portrays a rectangular polygonal body example.
FIG. 10A portrays a nebulous rectangular polygonal body example.
FIG. 11 shows a trapezoidal polygonal body embodiment within the scope of the current invention.
FIG. 11A shows a nebulous trapezoidal polygonal body embodiment within the ambit of the current invention.
FIG. 12 enables a hexagonal polygonal body example.
FIG. 12A enables a nebulous hexagonal polygonal body example.
FIG. 13 portrays an example of a cylindrical or elliptic cylindrical stabilizer (80).
FIG. 14 is a frontal perspective of stabilizer (80) where cover (60) is attached to extension (102).
FIG. 15 is a top plan view of a cylindrical body example.
FIG. 16 is a top plan view of an elliptic cylindrical body example.
FIG. 17 is a top plan view of a nebulous cylindrical-like body example.
FIG. 18 is a top plan view of an elliptic cylindrical-like body example.
FIG. 19 is an exemplification of the steps of an example of a method of using the stabilizer for assisting the stabilization of a spinal implant.
FIG. 20 is a diagrammatic representation of the steps of another example of a method of using the stabilizer for assisting the stabilization of a spinal implant.
FIG. 21 is another diagrammatic representation of the steps of still another example of a method of using the stabilizer for assisting the stabilization of a spinal implant.

### DESCRIPTION OF THE PREFERRED EMBODIMENTS

Although the disclosure hereof is detailed to enable those skilled in the art to practice the invention, the embodiments published herein merely exemplify the present invention.

In the most general sense, select preferred embodiments of the present invention include a cage or implant that can be inserted into a cavity of the spinal column. Surgical removal of mammalian spinal tissue in one or more spinal regions creates the cavity or cavities that will receive the implant or implants. It has been discovered that many embodiments of the current implant can be useful for spine surgeries and can assist in stabilizing injured, deformed and or degenerative spines. Preferred embodiments of the current invention can be employed with thoracic or lumbar spinal procedures. Still other preferred embodiments of the present invention are particularly suited for corpectomy or partial corpectomy procedures.

After insertion of the implant into the cavity, the biocompatible cage and the end cap or the end cap and connector combinations assist in stabilizing the spinal column against rotational movement and the combinations also resist the compression forces associated with gravity on the spinal column. It has been discovered that the biocompatible cage, end cap and connector combination is particularly useful for assisting in the restoration of normal anatomical height and angulation of an abnormal vertebral body. Select preferred embodiments of the present invention can be implanted through the patient's anterior or ventral side. The current spinal implants are custom fitted for the surgical cavity into which they will be inserted, i.e., the cage can be manufactured to fit the surgical cavity or the cage can be severed across a first cross section or the cage can be severed across first and second cross sections to size the cage to fit the cavity.

Preferred embodiments of the current biocompatible cage are generally trapezoidal in shape and are manufactured of titanium alloys, stainless steel, resorbable polymers, non-resorbable polymers or any other composition acceptable in the art. However, it has been discovered that end caps and/or connectors can be manufactured to be compatible with cages of generally square or rectangular dimensions.

Within the scope of the present invention, it has advantageously been discovered that the trapezoidal cages can have a width of from about six to about fifteen millimeters, as measured along the narrowest parallel of the trapezoid, and a depth of from about eight millimeters to about fifteen millimeters, as measured along a converging side of the trapezoid. Generally the spacing between dividers of the cage is from about five to about ten millimeters, as measured from outward side to outward side of the series of dividers. Openings of the cages of the current invention into which bone graft, osteogenic and/or arthrodesis accelerating substances are packed can have areas from about 36 millimeters² to 225 millimeters² or greater. Custom made biocompatible cages having lengths that range from about twelve millimeters to about seventy millimeters.

Preferred embodiments of the end caps of the present invention have dimensions that are compatible with biocompatible cage. In select preferred embodiments, the slides can extend away from the body of the end cap for distances of from about two millimeters to about ten millimeters. Embodiments of the end caps are provided with bores capable of engaging spikes that extend away from the biocompatible cage.

Preferred embodiments of the connectors of the current invention have legs with heights of about two millimeters and pins that extend about one and one-half millimeters beyond the legs. Anterior widths of the connectors range from about six millimeters to about fifteen millimeters. The anterior heights of the connectors are about seven millimeters. And depths of the connectors range from about six millimeters to more than fifteen millimeters.

Meeting a long felt but unfilled need in the spinal surgical arts, the unique structures of the present combinations allow the surgical team to, among other things, enhance the length of the spinal column from about two millimeters to about twenty millimeters more than the span of the biocompatible cage, when the patient's medical condition requires. Contact between the surgical cavity wall and the wedge-like cage can also inhibit the implant from contacting the spinal cord. It appears that having the apertures of select embodiments in such close proximity with the cavity's walls increases the probability of the osteogenic materials procuring a blood supply. And it is believed that increasing the blood supply to the osteogenic materials held by the cage enhances local areas of arthrodesis between the vertebra and the bone graft. Select preferred embodiments of the present invention are provided with lateral brakes to further impede the implant from contacting the spinal column.

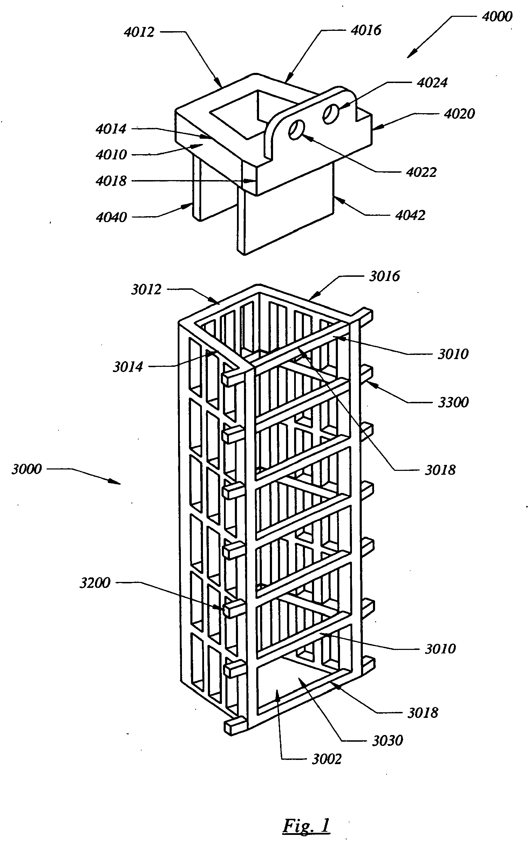

FIG. 1 is an exploded perspective enabling a biocompatible cage (3000) and end cap (4000) combination. Biocompatible cage (3000) has a series of trapezoidal dividers or braces (3010) and a plurality of receptacles (3002) that can be packed with osteogenic materials, arthrodesis accelerating substances or other substances prior to the completion of spinal surgery. Implant (3000) is manufactured of titanium, stainless steel, resorbable polymers, non-resorbable polymers or combinations thereof. By way of illustration, dividers or braces (3010) have inward sides (3012) (after insertion into the surgically created cavity, positioned near the dura mater of the spinal cord), outward side (3018) (proximate the surgeon after insertion into the surgically created cavity), first converging or lateral side (3014) and second converging or lateral side (3016). The combination of each inward side (3012), outward side (3018), first converging side (3014) and second converging side (3016) creates trapezoidal aperture (3030). Select preferred embodiments of biocompatible cage (3000) can be provided with one or more lateral brakes (3200, 3300).

An example of end cap (4000) is provided with implant facing border or boundary (4010) proximate implant (3000) and plate (4020) that, after insertion into the patient, is distal from biocompatible cage (3000), but proximate to the surgical team. In select preferred embodiments, border (4010) and plate (4020) are integral. Plate (4020) is provided with openings (4022, 4024) for receiving fasteners (not shown in this view). The fasteners, such as screws, are capable of securely attaching plate (4020) to bone. When the combination of implant (3000) and end cap (4000) are assembled as a unit, plate (4020) extends outward and away from border or boundary (4010) in a longitudinal direction parallel the lengthwise axis of biocompatible cage (3000). Boundary (4010) includes shortest side (4012), longest side (4018), first converging side (4014) and second converging side (4016). Extending from border (4010) toward cage (3000) are parallel docking slides (4040, 4042) for engaging inward side (3012) and outward side (3018) of outermost brace (3010) of implant (3000). As shown in FIG. 1, border (4010) is a four-side trapezoidal configuration..

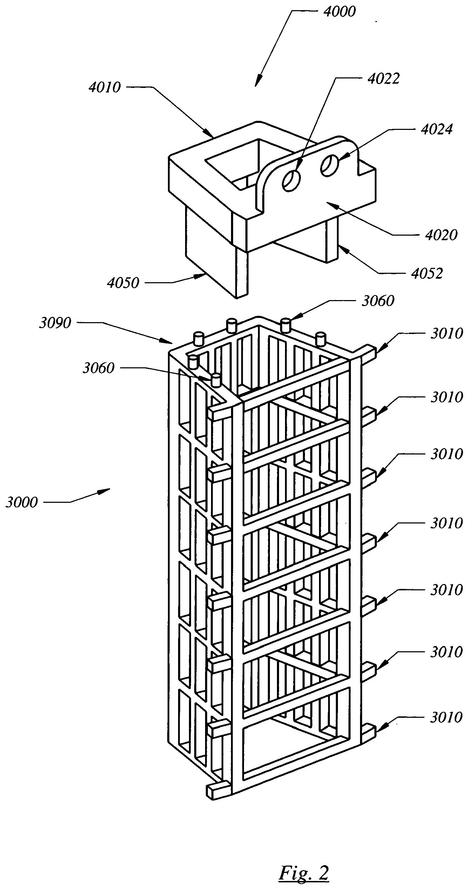

With reference to FIG. 2, a preferred embodiment of biocompatible cage (3000) that utilizes spikes (3060) is enabled. Except for spikes (3060) and the docking slides (4050 and 4052), the structural elements for the FIG. 2 implant (3000) are identical to the structures enabled by FIG. 1. As shown in FIG. 2, docking slides (4050 and 4052) are not parallel the other. Instead, docking slide (4050) is parallel first converging side (4014) of border (4010) for engaging first converging side (3014) of outermost brace (3010) of implant (3000) and docking slide (4052) is parallel second converging side (4016) of border (4010) for engaging second converging side of outermost brace (3010) of implant (3000).

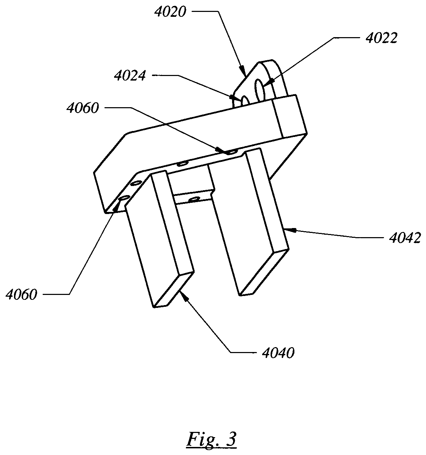

FIG. 3 is a perspective of a preferred embodiment of an implant facing side of border (4010) of end cap (4000). Along with docking slides (4040) and (4042), implant facing side of border (4010) is provided with bores (4060) capable of aligning with spikes (3060) of cage (3000).

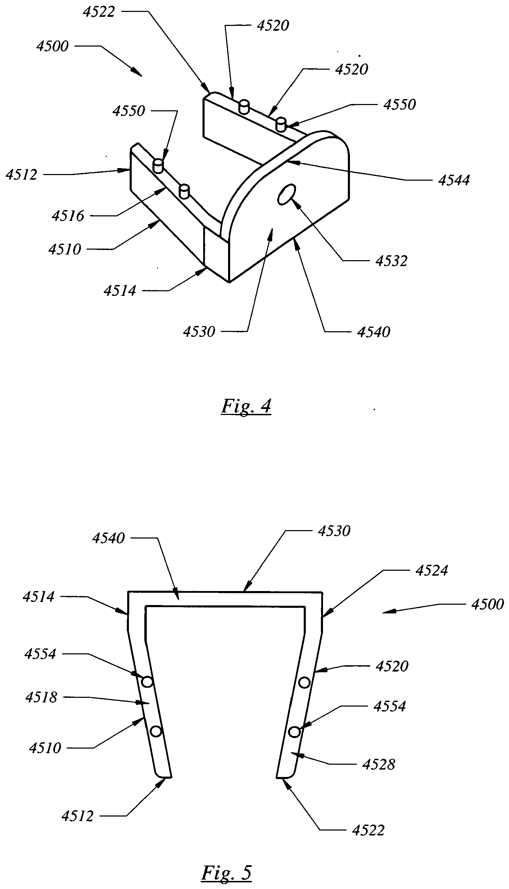

FIG. 4 is a perspective of a preferred embodiment of a connector or extension (4500) capable of being positioned between cage (3000) and end cap (4000). Connector (4500) has first leg (4510) and second leg (4520). As shown in FIGS. 4 and 5, distal end (4512) of first leg (4510) and distal end (4522) of second leg (4520) converge toward the other. However, within the scope of the present invention, when engineering parameters require, first and second legs (4510 and 4520) can be parallel the other or diverging from the other. First side (4516) of first leg (4510) is provided with pins (4550) and first side (4526) of second leg (4520) is provided with pins (4550). Pins (4550) extend in a lengthwise direction away from biocompatible cage (3000).

Anterior section (4530) of connector or extension (4500) joins a first end (4514) of first leg (4510) and a second end (4524) of second leg (4520). Anterior section (4530), first leg (4510) and second leg (4520) share a coplanar margin (4540) that faces implant (3000). Anterior section (4530) is provided with a second margin (4544) opposite coplanar margin (4540) that extends in a longitudinal direction further away from biocompatible implant (3000) than pins (4550). In select preferred embodiments of extension (4000), anterior section (4530) includes hole (4532) capable of being engaged by a surgical tool (not shown in this view).

FIG. 5 is a plan view of coplanar margin (4540) of connector (4500). Second side (4518) of first leg (4510) and second side (4528) of second leg (4520) are provided with apertures (4554) capable of receiving spikes (3060) of biocompatible cage (3000).

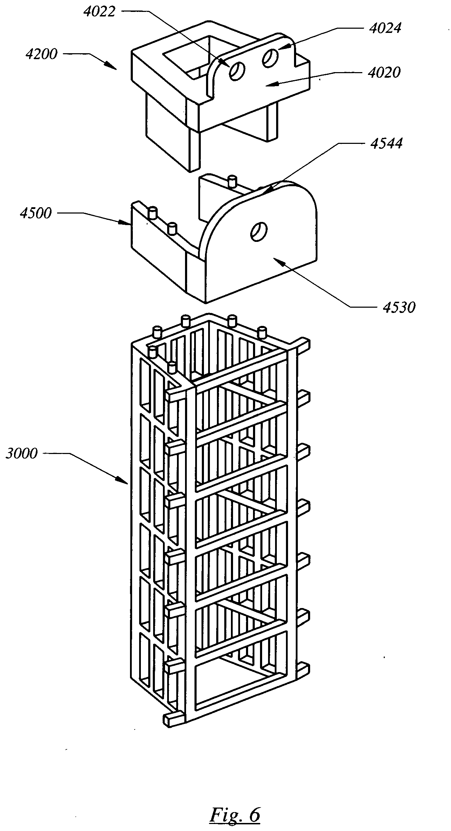

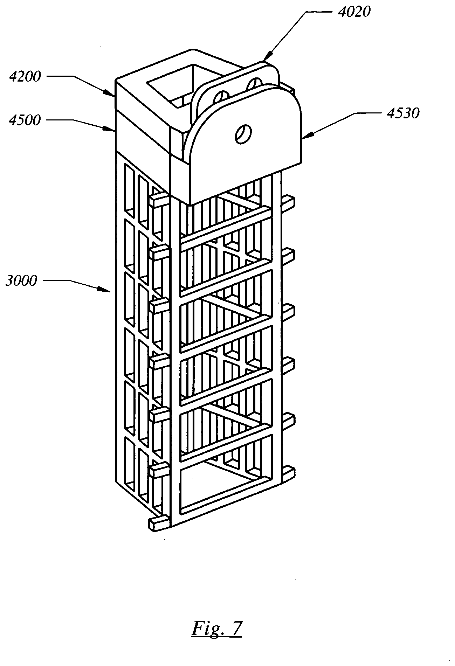

FIG. 6 is an exploded perspective showing a preferred embodiment of the combination of biocompatible cage (3000), connector or extension (4500) and end cap (4200). FIG. 7 is a perspective showing a preferred embodiment of an engaged combination of biocompatible cage (3000), connector or extension (4500) and end cap (4200). When biocompatible cage (3000), connector (4500) and end cap (4200) are fully engaged within the surgical cavity, anterior section (4530) of connector (4500) is anterior to plate (4020) of end cap (4200). Second margin (4544) and anterior section (4530) of connector (4500) sufficiently cover openings (4022 and 4024) to prevent fasteners (not shown in this view) from backing out of plate (4020) of end cap (4200).

The practice of select preferred embodiments of the present stabilizer requires the removal of mammalian tissue to create a cavity for receiving a spinal implant. Depending on the surgical procedure performed, spinal implants of varying sizes and shapes may be selected for eventual implantation into the surgical cavity. Preferred embodiments of the current stabilizer are manufactured in differing sizes and shapes and are compatible with numerous spinal implants of amorphous or geometrically defined spinal supporting structures.

In the most general sense, the current invention is a stabilizer for assisting with the stabilization of a spinal implant that will be implanted into the surgically created cavity. Stabilizers in accord with the present invention can have polygonal or polygonal-like bodies. In other words, when engineering parameters require, the polygonal-like bodies can include one or more nebulous segments modifying the standardized geometric polygonal bodies. Thus, select preferred embodiments of the stabilizer can be engineered to have a socket for fitting a spinal implant of virtually any outward supporting structure dimension. Unless otherwise indicated, for the remainder of this specification: the term "polygonal" body can also refer to a polygonal-like body; the term "cylindrical" body can also refer to a cylindrical-like body; and the term "elliptic cylindrical" body can also refer to an elliptic cylindrical-like body.

For preferred embodiments of the stabilizer, an anterior wall or extension of the body includes a plurality of apertures and extends beyond an outward edge of the polygonal body. Each stabilizer further includes a cover that has an aperture that can be aligned with one of the plurality of apertures positioned on the body's anterior wall or extension. Fasteners secure the cover to the polygonal body as well as bone. After attachment to the polygonal body, the cover is capable of preventing egress of fasteners securing the stabilizer to bone. Stabilizers of the current invention can be manufactured of biocompatible metals, plastics or combinations thereof, and preferred embodiments are manufactured of titanium, titanium alloys, stainless steel, non-resorbable and resorbable polymers. The current stabilizer meets the long felt but unfilled need of providing a stabilizer for use with a spinal implant that has a polygonal shape, where the stabilizer is attached to bone at first end of the stabilizer and has a socket at the second end for connecting to a lengthwise end of the spinal implant.

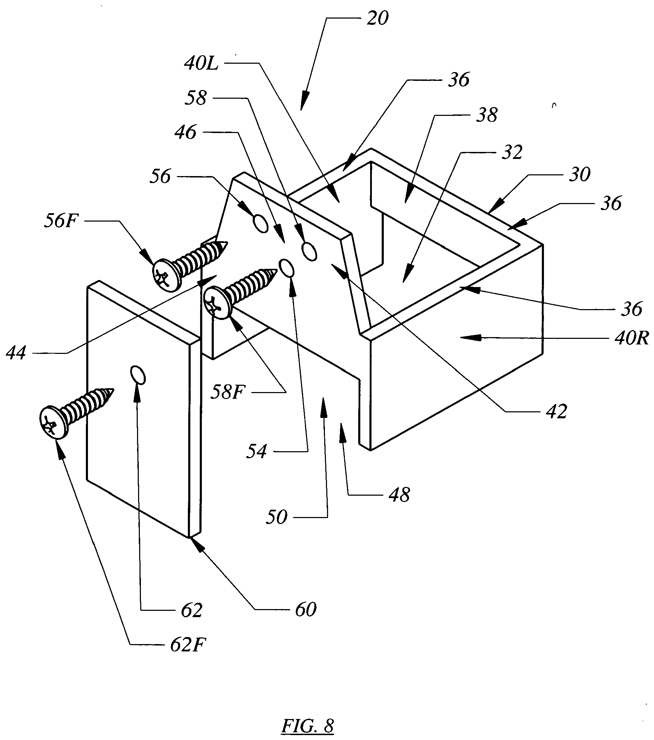

FIG. 8 is an exploded view frontal perspective of an example of the stabilizer (20) having a polygonal body (30) and attachable cover (60). Polygonal body (30) encloses opening or cavity (32). Perimeter (36) is distal from spinal implant (not shown in this view). Extending from perimeter (36) toward spinal implant are posterior wall (38) and lateral walls (40L and 40R). Posterior wall (38) and lateral walls (40L and 40R) extend approximately identical lengths from perimeter (36). Anterior wall (42) has first section (44) extending from perimeter (36) toward the spinal implant and second section (46) extending from perimeter (36) away from the spinal implant. Since first section (44) is of lesser length than posterior wall (38) and lateral walls (40L and 40R), socket (50) for fitting about a lengthwise end of the spinal implant is created. A lengthwise end of the spinal implant can be fitted through gap (48) into socket (50). Second section (46) of anterior wall (42) is provided with apertures (54, 56 and 58). In select preferred embodiments, a portion of anterior wall (42) extends beyond perimeter (36) in a direction away from the spinal implant. As shown in FIG. 1, perimeter (36) is open to cavity (32), but in select preferred embodiments perimeter (36) can be a continuous surface such that one end of cavity (32) is closed.

Cover (60) is provided with aperture (62) that upon attachment to polygonal body (30) aligns with aperture (54) of second section (46) of anterior wall (42). When cover (60) is attached to polygonal body (30), cover (60) prevents egress of fasteners (56F and 58F) from second section (46) of polygonal body (30). Fastener (62F) secures cover (60) at aperture (54) to second section (46) such that cover (60) blocks egress of the spinal implant (not shown in this view) from gap (48) and socket (50).

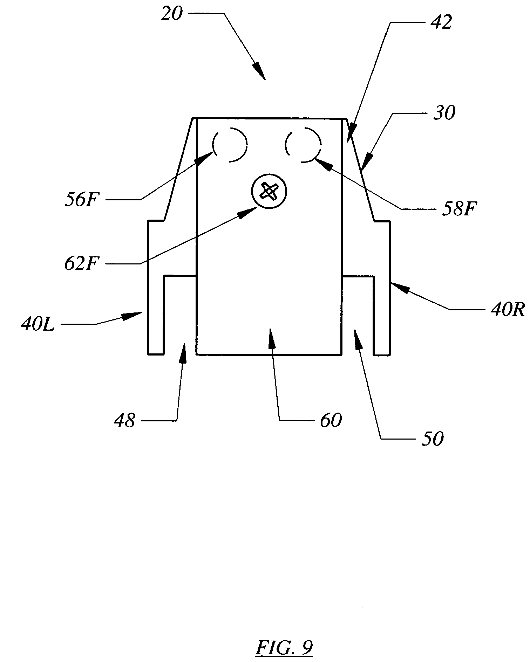

FIG. 9 is a frontal view of stabilizer (20) where cover (60) is attached to anterior wall (42) of polygonal body (30). Fastener (62) secures cover (60) to anterior wall to block egress of fasteners (56F and 58F) from apertures (56 and 58), respectively. Cover (60) is of sufficient length to block egress of the spinal implant (not shown in this view) from gap (48) and socket (50).

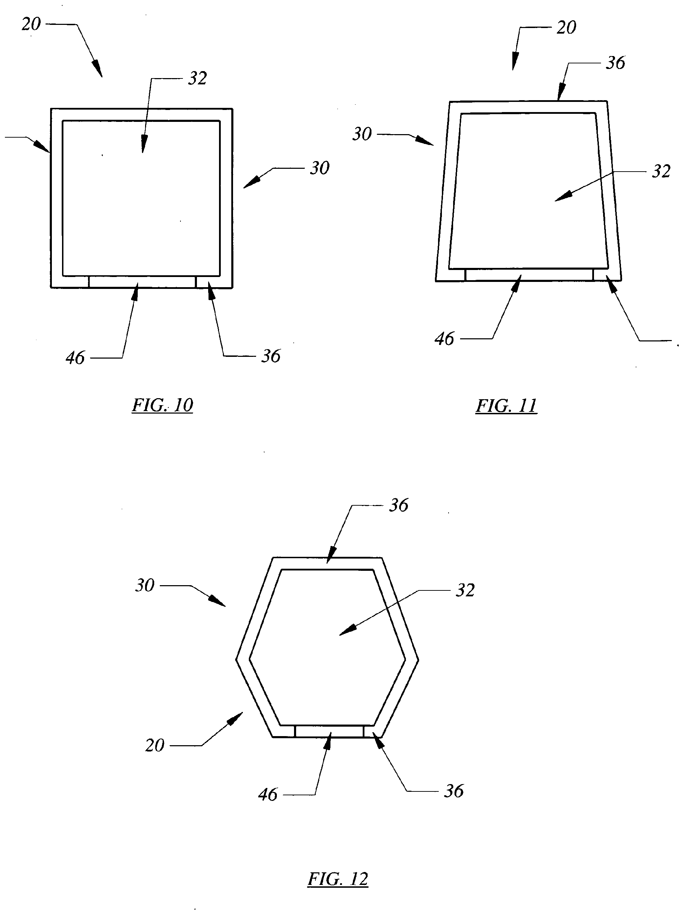

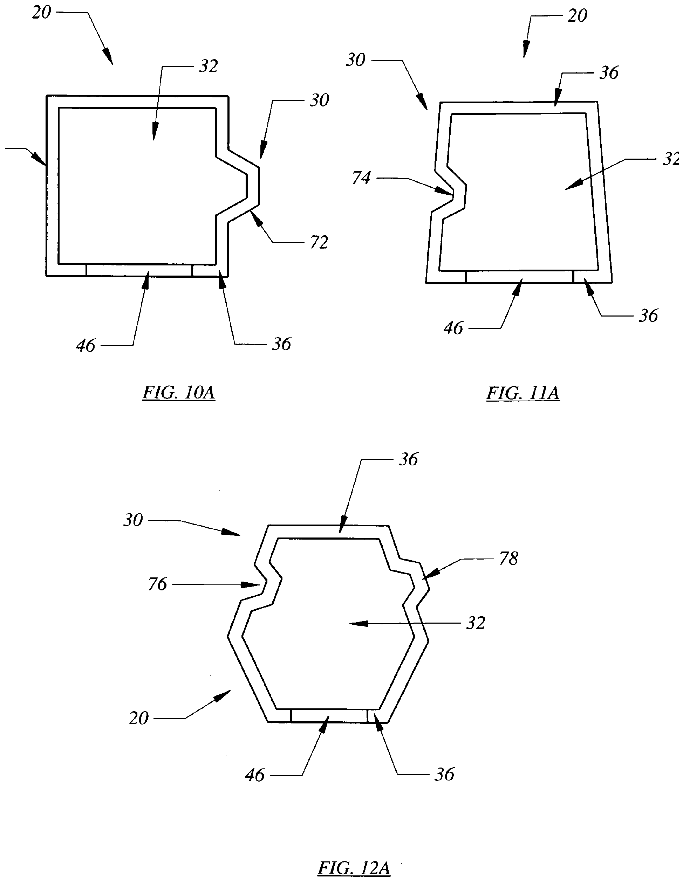

By way of illustration and not limitation, FIGS. 10-12 are top plan views of preferred embodiments or examples of stabilizers (20) with different shaped polygonal bodies (30). FIG. 10 portrays a rectangular polygonal body (30). FIG. 11 shows a trapezoidal polygonal body (30). FIG. 12 enables a hexagonal polygonal body (30). Although not shown in FIGS. 10-12, depending on engineering parameters and the design of the spinal implant that will be fitted into the stabilizer's socket, polygonal stabilizers of other shapes are within the scope of the present invention.

In the preferred embodiments or examples displayed in FIG. 10-12, openings or cavities (32), perimeters (36) and second sections (46) of anterior walls (42) are disclosed. For select preferred embodiments not shown in FIGS. 10-12, second sections (46) can be the depth of perimeter (36). In other words, second sections (46) of anterior walls (42) equate with the perimeters for depth and width of second sections (46).

By way of illustration and not limitation, FIGS. 10A-12A are top plan views of preferred embodiments or examples of stabilizers (20) with amorphous or nebulous shaped polygonal-like bodies (30). FIG. 10A portrays one of a plethora of nebulous rectangular-like polygonal bodies (30) having amorphous segment (72). FIG. 11A shows one of many potential nebulous trapezoidal-like polygonal bodies (30) including amorphous segment (74). FIG. 12A enables one or a plethora of hexagonal-like polygonal bodies (30) having amorphous segments (76 and 78).

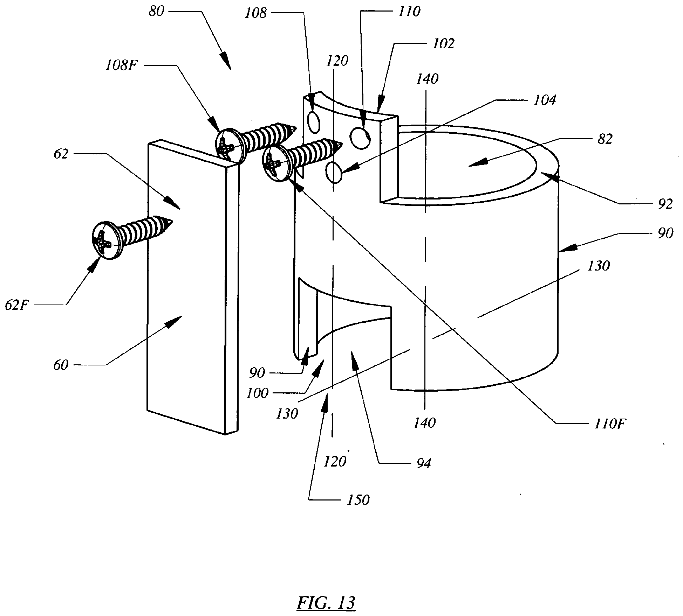

FIG. 13 portrays an example of a cylindrical or elliptic cylindrical stabilizer (80). Cylindrical or elliptic cylindrical wall (90) encloses lumen or cavity (82). Rim (92) is distal from the spinal implant (not shown in this view). Gap (94) of cylindrical or elliptic cylindrical wall (90) is distal from rim (92). Gap (94) in cylindrical or elliptic cylindrical wall (90) creates socket (100) for fitting about a lengthwise end of the spinal implant.

Extension (102) extends beyond rim (92) in a direction away from the spinal implant. In select preferred embodiments, extension (102) is proximate gap (94). Extension (102) is provided with apertures (104, 108 and 110). In select preferred embodiments, extension (102) follows the contour of rim (92). In still other select preferred embodiments, extension (102) is coplanar with central lengthwise axis (120-120) that intersects center (150) of gap (94) and is simultaneously perpendicular to axis (130-130) that is simultaneously perpendicular to central longitudinal axis (140-140) wherein axis (130-130) intersects center (150) of gap (94). As shown in FIG. 6, cavity (82) is open to rim (92), but in other select preferred embodiments a continuous surface covers cavity (82) to create an end of stabilizer (80) that is enclosed rather than open.

Cover (60) is provided with aperture (62) that upon attachment to extension (102) of stabilizer (80) corresponds to aperture (104) of extension (102). When cover (60) is attached to stabilizer (80), cover (60) prevents egress of fasteners (108F and 110F) from extension (102) of stabilizer (80). Fastener (62F) secures cover (60) at aperture (104) of extension (102) such that cover (60) blocks egress of the spinal implant (not shown in this view) from gap (94) and socket (100).

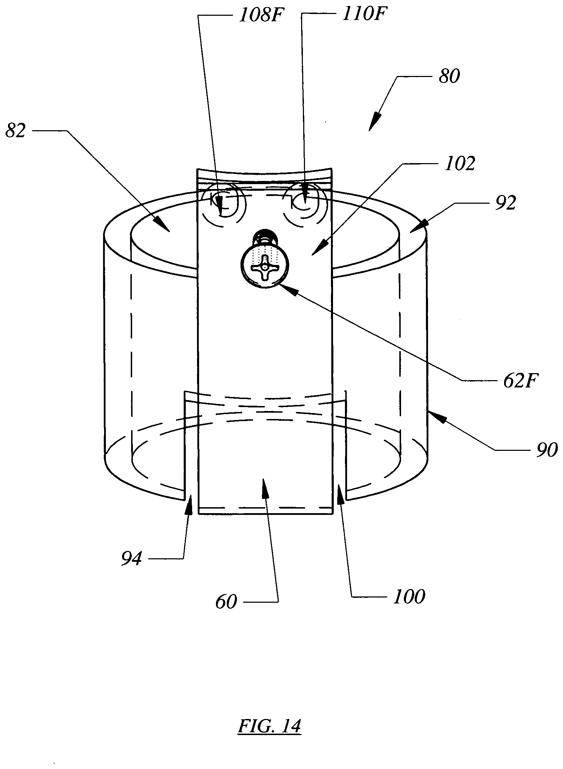

FIG. 14 is a frontal perspective of stabilizer (80) where cover (60) is attached to extension (102). Fastener (62) secures cover (60) to stabilizer (80) to block egress of fasteners (108F and 110F) from apertures (108 and 110), respectively. Cover (60) is of sufficient length to block egress of the spinal implant (not shown in this view) from gap (94) and socket (100).

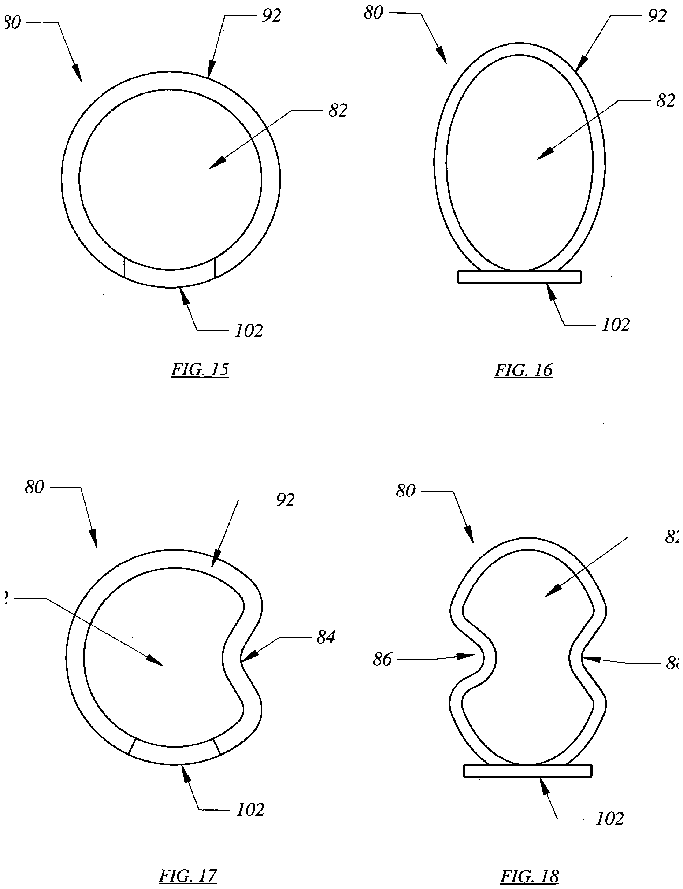

By way of illustration and not limitation, FIGS. 15-18 are top plan views of preferred embodiments of cylindrical or elliptic cylindrical stabilizer (80). FIG. 15 portrays cylindrical body (80) including lumen (82), rim (92) and extension (102). FIG. 16 shows an elliptic cylindrical body (80) having lumen (82), rim (92) and extension (102). FIG. 17 enables one of a plethora of nebulous cylindrical-like bodies (80) having lumen (82), amorphous segment (84), rim (92) and extension (102). FIG. 18 shows one of a plethora of nebulous elliptic cylindrical-like bodies including lumen (82), amorphous segments (86 and 88), rim (92) and extension (102).

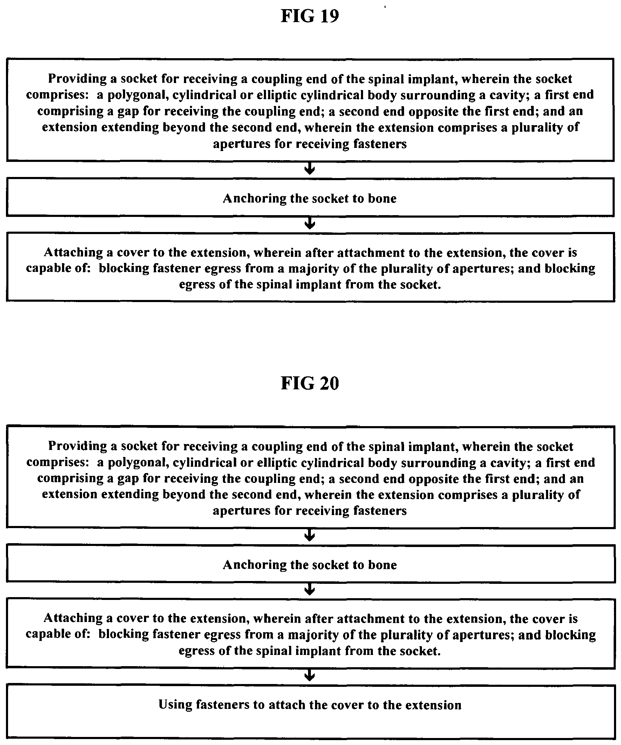

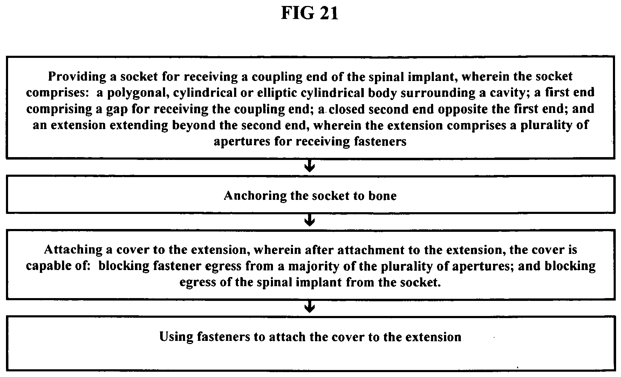

Steps associated with the practice of the methods of embodiments the present invention are set forth in FIGS. 19-21. Those steps are related to the practice of using the stabilizer structures previously set forth.

Having disclosed the spinal implant, Applicant now prays respectfully that a patent be granted for its invention in accordance with the scope of the claims appended hereto.

## Claims

1. A combination (3000, 4000) capable of insertion about a surgically created cavity proximate to one or more vertebrae; said combination (3000, 4000) comprising:
a) a biocompatible cage (3000) comprising:
i) a length having a longitudinal axis;
ii) a first end perimeter (3010) at a first end of said biocompatible cage (3000);
b) an end cap (4000) comprising:
i) a border (4010) comprising four sides (4012, 4014, 4016, 4018) and
ii) a plate (4020) extending in said lengthwise direction parallel said longitudinal axis away from said biocompatible cage (3000) and comprising at least one opening (4022, 4024) for receiving a fastener; **characterized in that**
c) the first end perimeter (3010) at the first end of said biocompatible case (3000) creates a trapezoidal aperture (3030);
d) the end cap (4000) further comprisesa pair of opposed docking slides (4050, 4052) extending from said border (4010) in said lengthwise direction toward said biocompatible cage (3000) and capable of docking with said first end perimeter (3010) of said biocompatible cage (3000);
e) said biocompatible cage (3000) further comprises a plurality of spikes (3060) extending outwardly from said first end perimeter (3010) in said lengthwise direction; and
f) said border (4010) of said end cap (4000) further comprises a first plurality of bores capable (4060) of aligning with some or all of said plurality of spikes (3060).

2. The combination of claim 1 further comprising: a connector (4500), capable of receiving said docking slides (4050, 4052) and positioned between said biocompatible cage (3000) and said end cap (4000); said connector (4500) comprising:
a) a first leg (4510) comprising:
i) a plurality of apertures (4554) capable of aligning with some of said plurality of spikes (3060); and
ii) a plurality of pins (4550) capable of aligning with some of said plurality of bores (4060); and
b) a second leg (4520) comprising:
i) a plurality of apertures (4554) capable of aligning with some of said plurality of spikes (3060); and
ii) a plurality of pins (4550) capable of aligning with some of said plurality of bores (4060).

3. The combination of claim 2, wherein said connector (4500) comprises an anterior section (4530) joining a first end (4514) of said first leg (4510) and a first end (4524) of said second leg (4520), wherein said anterior section (4530) shares a margin facing said biocompatible cage (3000) with said legs (4510, 4520) and includes an opposing margin extending further away from said biocompatible cage (3000) than said pluralities of pins (4550), and wherein said anterior section (4530) is anterior to said plate (4020).

4. The combination (3000, 4000) of claim 1 wherein each of said docking slides (4040,4042) is parallel its opposite docking slide.

5. The combination of claims 1 to 4, wherein said biocompatible cage (3000) comprises one or more lateral brakes (3200, 3300).

6. The combination of claims 1 to 5, wherein said first leg (4510) and said second leg (4520) are parallel.

7. The combination of claims 1 to 6, wherein said first leg (4510) and said second leg (4520) converge toward each other as said first leg (4510) and said second leg (4520) advance away from said anterior section (4530).

8. The use of an end cap (4000) in the combination according to one of the claims 1 to 7.

9. The use of a biocompatible cap (3000) in the combination according to one of the claims 1 to 7.

10. The use of a connector (4500) in the combination according to one of the claims 2or 3 or in the combination according to one of the claims 4 to 7 as also related to claim 2.

## Patentansprüche

1. Kombination (3000, 4000), die zum Einsetzen um einen chirurgisch erzeugten Hohlraum nahe einem oder mehreren Wirbeln in der Lage ist, wobei die Kombination (3000, 4000) Folgendes umfasst:
a) einen biologisch verträglichen Korb (3000), der Folgendes umfasst:
i) eine Länge, die eine Längsachse hat,
ii) eine erste Umfassung (3010) an einem ersten Ende des biologisch verträglichen Korbes (3000),
b) eine Endkappe (4000), die Folgendes umfasst:
i) eine Umrandung (4010), die vier Seiten (4012, 4014, 4016, 4018) umfasst, und
ii) eine Platte (4020), die sich in der Längsrichtung parallel zu der Längsachse von dem biologisch verträglichen Korb (3000) weg erstreckt und wenigstens eine Öffnung (4022, 4024) zum Aufnehmen eines Befestigungselements umfasst, **dadurch gekennzeichnet, dass**
c) die erste Umfassung (3010) an dem ersten Ende des biologisch verträglichen Korbes (3000) eine trapezförmige Öffnung (3030) schafft,
d) die Endkappe (4000) ferner ein Paar von gegenüberliegenden Andockgleitern (4050, 4052) umfasst, die sich von der Umrandung (4010) aus in der Längsrichtung zu dem biologisch verträglichen Korb (3000) hin erstrecken und zum Andocken mit der ersten Umfassung (3010) des biologisch verträglichen Korbes (3000) in der Lage sind,
e) der biologisch verträgliche Korb (3000) ferner mehrere Dorne (3060) umfasst, die sich von der ersten Umfassung (3010) aus in der Längsrichtung nach außen erstrecken, und
f) die Umrandung (4010) der Endkappe (4000) ferner mehrere Bohrungen (4060) umfasst, die zum Ausrichten mit einigen oder allen der mehreren Dorne (3060) in der Lage sind.

2. Kombination nach Anspruch 1, die ferner Folgendes umfasst: einen Verbinder (4500), der zum Aufnehmen der Andockgleiter (4050, 4052) in der Lage und zwischen dem biologisch verträglichen Korb (3000) und der Endkappe (4000) angeordnet ist, wobei der Verbinder (4500) Folgendes umfasst:
a) einen ersten Schenkel (4510), der Folgendes umfasst:
i) mehrere Öffnungen (4554), die zum Ausrichten mit einigen der mehreren Dorne (3060) in der Lage sind, und
ii) mehrere Stifte (4550), die zum Ausrichten mit einigen der mehreren Bohrungen (4060) in der Lage sind,
b) einen zweiten Schenkel (4520), der Folgendes umfasst:
i) mehrere Öffnungen (4554), die zum Ausrichten mit einigen der mehreren Dorne (3060) in der Lage sind, und
ii) mehrere Stifte (4550), die zum Ausrichten mit einigen der mehreren Bohrungen (4060) in der Lage sind.

3. Kombination nach Anspruch 2, wobei der Verbinder (4500) eine anteriore Sektion (4530) umfasst, die sich an ein erstes Ende (4514) des ersten Schenkels (4510) und an ein erstes Ende (4524) des zweiten Schenkels (4520) anschließt, wobei die anteriore Sektion (4530) einen Rand, der zu dem biologisch verträglichen Korb (3000) zeigt, mit den Schenkeln (4510, 4520) teilt und einen gegenüberliegenden Rand einschließt, der sich weiter weg von dem biologisch verträglichen Korb (3000) erstreckt als die mehreren Stifte (4550), und wobei die anteriore Sektion (4530) anterior zu der Platte (4020) liegt.

4. Kombination (3000, 4000) nach Anspruch 1, wobei jeder der Andockgleiter (4050, 4052) parallel zu seinem gegenüberliegenden Andockgleiter ist.

5. Kombination nach einem der Ansprüche 1 bis 4, wobei der biologisch verträgliche Korb (3000) eine oder mehrere seitliche Bremsen (3200, 3300) umfasst.

6. Kombination nach einem der Ansprüche 1 bis 5, wobei der erste Schenkel (4510) und der zweite Schenkel (4520) parallel sind.

7. Kombination nach einem der Ansprüche 1 bis 6, wobei der erste Schenkel (4510) und der zweite Schenkel (4520) zueinander hin zusammenlaufen, wenn der erste Schenkel (4510) und der zweite Schenkel (4520) von der anterioren Sektion (4530) weg fortschreiten.

8. Verwendung einer Endkappe (4000) in der Kombination nach einem der Ansprüche 1 bis 7.

9. Verwendung einer biologisch verträglichen Kappe (3000) in der Kombination nach einem der Ansprüche 1 bis 7.

10. Verwendung eines Verbinders (4500) in der Kombination nach einem der Ansprüche 2 oder 3 oder in der Kombination nach einem der Ansprüche 4 bis 7, sofern ebenfalls mit Anspruch 2 verknüpft.

## Revendications

1. Combinaison (3000, 4000) capable d'insertion sur une cavité créée de manière chirurgicale à proximité d'une ou plusieurs vertèbre(s), ladite combinaison (3000, 4000) comprenant :
a) une cage biocompatible (3000) comprenant :
i) une longueur ayant un axe longitudinal ;
ii) un périmètre de première extrémité (3010) sur une première extrémité de ladite cage biocompatible (3000) ;
b) un capuchon d'extrémité (4000) comprenant :
i) une bordure (4010) comprenant quatre côtés (4012, 4014, 4016, 4018) et
ii) une plaque (4020) s'étendant dans ladite direction en longueur parallèlement audit axe longitudinal en s'éloignant de ladite cage biocompatible (3000) et comprenant au moins une ouverture (4022, 4024) pour recevoir une fixation ; **caractérisé en ce que**
c) le périmètre de première extrémité (3010) sur la première extrémité de ladite cage biocompatible (3000) crée une ouverture trapézoïdale (3030) ;
d) le capuchon d'extrémité (4000) comprend en outre une paire de guides d'arrimage opposés (4050, 4052) s'étendant à partir de ladite bordure (4010) dans ladite direction en longueur en direction de ladite cage biocompatible (3000) et capables d'arrimage avec ledit périmètre de première extrémité (3010) de ladite cage biocompatible (3000) ;
e) ladite cage biocompatible (3000) comprend en outre une pluralité de crampons (3060) s'étendant vers l'extérieur à partir dudit périmètre de première extrémité (3010) dans ladite direction en longueur ; et
f) ladite bordure (4010) dudit capuchon d'extrémité (4000) comprend en outre une première pluralité d'alésages capables (4060) d'alignement avec toute ou partie de ladite pluralité de crampons (3060).

2. Combinaison selon la revendication 1 comprenant en outre : un connecteur (4500) capable de recevoir lesdits crampons d'arrimage (4050, 4052) et positionné entre ladite cage biocompatible (3000) et ledit capuchon d'extrémité (4000) ; ledit connecteur (4500) comprenant :
a) une première branche (4510) comprenant :
i) une pluralité d'ouvertures (4554) capables d'alignement avec une partie de la pluralité de crampons (3060) ; et
ii) une pluralité de broches (4550) capables d'alignement avec une partie de ladite pluralité d'alésages (4060) ; et
b) une seconde branche (4520) comprenant :
i) une pluralité d'ouvertures (4554) capables d'alignement avec une partie de ladite pluralité de crampons (3060) ; et
ii) une pluralité de broches (4550) capables d'alignement avec une partie de ladite pluralité d'alésages (4060).

3. Combinaison selon la revendication 2, dans laquelle ledit connecteur (4500) comprend une section antérieure (4530) joignant une première extrémité (4514) de ladite première branche (4510) et une première extrémité (4524) de ladite seconde branche (4520), dans laquelle ladite section antérieure (4530) partage une marge faisant face à ladite cage biocompatible (3000) avec lesdites branches (4510, 4520) et inclut une marge opposée s'étendant plus loin de ladite cage biocompatible (3000) que lesdites pluralités de broches (4550) et dans laquelle ladite section antérieure (4530) est antérieure à ladite plaque (4020).

4. Combinaison (3000, 4000) selon la revendication 1, dans laquelle chacun desdits crampons d'arrimage (4040, 4042) est parallèle à son crampon d'arrimage opposé.

5. Combinaison selon les revendications 1 à 4, dans laquelle ladite cage biocompatible (3000) comprend un ou plusieurs freins latéraux (3200, 3300).

6. Combinaison selon les revendications 1 à 5, dans laquelle ladite première branche (4510) et ladite seconde branche (4520) sont parallèles.

7. Combinaison selon les revendications 1 à 6, dans laquelle ladite première branche (4510) et ladite seconde branche (4520) convergent l'une vers l'autre à mesure que ladite première branche (4510) et ladite seconde branche (4520) avancent en s'éloignant de ladite section antérieure (4530).

8. Utilisation d'un capuchon d'extrémité (4000) dans la combinaison selon l'une des revendications 1 à 7.

9. Utilisation d'un capuchon biocompatible (3000) dans la combinaison selon l'une des revendications 1 à 7.

10. Utilisation d'un connecteur (4500) dans la combinaison selon l'une des revendications 2 ou 3 ou dans la combinaison selon l'une des revendications 4 à 7 comme également en lien avec la revendication 2.
